# EUROPEAN PATENT SPECIFICATION

(11) **EP 0 228 466 B1**
(45) Date of publication and mention of the grant of the patent: **30.08.1995**
(21) Application number: 86904697.9
(22) Date of filing: 14.07.1986
(51) Int. Cl.: A61M 1/00, F16T 1/00

(54) **FLUID-FLOW CONTROLLING DEVICE AND APPARATUS EMPLOYING SAME**
DURCHFLUSSREGLER SOWIE DENSELBEN ENTHALTENDE VORRICHTUNG
DISPOSITIF DE COMMANDE DE L'ECOULEMENT D'UN FLUIDE ET APPAREIL L'UTILISANT

(30) Priority: 16.07.1985 US 755428
(43) Date of publication of application: 15.07.1987
(73) Proprietor: GENERAL POLYMERIC CORPORATION, West Reading, PA 19611 (US)
(72) Inventor: FERRI, Joseph, E., Shillington, PA 19607 (US)
(74) Representative: Gillam, Francis Cyril
(86) International application number: US8601502
(87) International publication number: WO8700439

(56) References cited:
- US-A- 3 661 815
- US-A- 3 898 143
- US-A- 3 982 538
- US-A- 4 076 663
- US-A- 4 228 798
- US-A- 4 328 828
- US-A- 4 465 485

## Description

### Field of the Invention

This invention relates to a device which is normally pervious to the flow of fluids through it, but which when it is contacted by a liquid becomes impervious to the flow of fluids. It relates also to the preparation of such a device and to apparatus utilizing the same, particularly in connection with the aspiration of body fluids from the site of a body wound.

Such a device, which normally permits the ready flow of gas through it, but which when contacted by liquid becomes substantially impervious to the flow of gas or liquid, has many applications. However, ii will be described hereinafter in detail with particular relation to apparatus for aspirating body fluids from the site of a body wound, for example, during or after surgical operation. As is known, during and after a surgical operation, various liquids tend to accumulate at the site of the operation, especially blood, but also other body fluids such as: lymphatic fluid, glandular fluid, fluids applied for medical purposes, etc. To aid in removing such fluids, it is common to provide an aspirating system comprising a flexible aspirating tube, the open free end of which is applied to the region to be cleared of fluids and the other end of which is supplied with a vacuum, so that the liquids are drawn into the tube and removed from the surgical site by much the same basic principle as is employed in an ordinary vacuum cleaner. Further, after the operation is over, it is common to maintain suction-type drains for continuously removing body fluids from the operation site.

In such a system, it is commonly desirable, and in some cases very important that the liquids so aspirated be collected locally in a suitable receptacle, so that the aspirated contents of the receptacle can be analyzed and/or disposed of in a sanitary manner. To this end, it is known to utilize a collecting container which is substantially airtight except for an air inlet and an air outlet (see, for example, U.S. Patent No. 3,704,709). The air inlet is connected to the end of an aspirating tube remote from the end at which aspiration of fluids from the patient occurs, and the outlet is connected to another tube which leads to the source of vacuum. Both inlet and outlet, openings are positioned well above the bottom of the container. Accordingly, liquids or other materials aspirated into the inlet in response to the action of the vacuum source fall to the bottom of the receptacle and are collected therein.

In such arrangement, it is of practical importance that the level of the liquid not be permitted to rise as high as the outlet opening. If the liquid level rises to the air outlet opening, liquid will begin to leave the collecting receptacle and flow into the vacuum line. This is generally undesirable for a number of reasons; first, if the vacuum line is connected directly to the vacuum source, such suction of body liquids into it may contaminate and/or interfere with its proper operation over a period of time, and if as is often the case, a mechanical valve is included in the suction line leading to the vacuum source to permit ready control of the connection of the vacuum source to the outlet of the collecting receptacle, spillage of the collected liquid into that valve may also interfere with its proper operation. In any event, such overflow from the container to the vacuum source will contaminate the vacuum source with whatever contaminants are present in the collected liquid. In addition, in many cases it is desired not to lose any liquid collected in the receptacle since it may be important to examine or analyze all of the liquid collected.

It is also desirable in many cases to filter the air sucked through the receptacle during normal operation, before it reaches the vacuum source, so as to prevent contamination of the vacuum source and the line leading to it with living contaminants such as bacteria, for example. Similarly, it is desirable also in certain situations to filter air that tends to be sucked into the collecting receptacle after the vacuum is terminated and as the vacuum tube between the source of the vacuum and the air outlet is opened or disengaged. Such filtering is designed to prevent contamination of the contents of the receptacle by contaminants such as bacteria, for example.

### Reported Developments

In an effort in accomplish the foregoing, it is known to use a device which is secured to the underside of the lid closing tile upper opening of the collecting receptacle and which senses the level of the collected liquid and terminates aspiration when a predetermined level somewhat lower than the outlet opening has been achieved. It is also known to include on the underside of the receptacle lid conventional porous or semi-porous filtering elements to prevent the passage of small amounts of the collected liquid into the vacuum source and to remove certain types and sizes of bacteria or other living contaminants.

One type of device previously known and utilized for sensing the level of the liquid in the receptacle comprises in essence a float valve having a floatable element which floats at the surface of the collected liquid, at least when the receptacle is nearly filled. The float valve responds to raising of the liquid level beyond a predetermined desired position by closing off the inlet or outlet air openings, or both. An example of an aspirating system which includes the use of a float valve is disclosed in U.S. Patent No. 3,680,560.

Such arrangements, involving rather sensitive constructions which have critical moving elements, are relatively expensive to make and to maintain, particularly in view of the environment in which they operate. Inasmuch as it is often desirable to discard, or at least sterilize, everything in the receptacle, the possible inconvenience and cost of such arrangements will be apparent. This is particularly true in view of the fact that the presently most desired type of apparatus is one which can economically be completely discarded after each use, and in which the lid is therefore permanently secured to the receptacle and not designed for removal and reuse. In such cases in which the lid, receptacle, and the entire contents are discarded, the importance of an inexpensive arrangement for accomplishing the desired termination of the aspirating and receptacle filling operation is very apparent.

Another problem that has been encountered in an arrangement which involves the use of a float valve is that the valve tends to be activated prematurely as a result of contact with foam or froth that collects in the receptacle. Blood, for example, is a natural frothing agent.

With respect to the use of heretofore known porous or semi-porous filtering elements which are affixed to the underside of the receptacle lid, and which function as both a valve and filter, such elements are generally made from hydrophobic polymeric materials such as, for example, polytetrafluoroethylene, polyester, poly(vinyl chloride), polypropylene, and polyethylene. Filter-valves made from such materials are disclosed in U.S. Patent No. 4,465,485. Experience has shown that, in operational procedures which utilize laser and/or electrical surgical devices, the smoke which emanates from the use of such devices tends to clog the filter-valve. Such clogging can substantially reduce the flow of air or even cut it off prematurely. In an attempt to overcome this problem, the aspirating system has been provided with a pre-filter to remove from the air, prior to its contact with the filter-valve, the smoke particles which cause the clogging. Polytetrafluoroethylene has also been used to form the pre-filter. Such a pre-filtering and filter-valve arrangement tends to be rather costly.

Another type of valve that has been described for use in apparatus for aspirating body fluids from the site of a body wound and in other types of apparatus, for example, a pipette, is disclosed in U.S. Patent No. 3,982,538. The valve disclosed in this patent comprises a container which is positioned in the path of liquid flow and which contains a powdered or granular, water soluble, polymeric material which permits the passage there through of air, but which, upon being contacted with an aqueous liquid, swells and blocks the passage there through of both liquid and air. The swelling of the water soluble polymeric material imposes limitations on the effective use of such a valve in various types of applications. In addition, the valve does not function effectively when used in applications where the vacuum pressure is relatively high, as is desired in many types of applications. Also, the valve does not function effectively as a filter for relatively small size solids.

In EP-0 066 702-A2, there is described a blood collector device having an automatic valving element which contains a water insoluble but water-swellable polymeric material. The valving element is located in a bore through a stopper, so as to close that bore on being contacted by blood. The valving element of this prior specification suffers similar disadvantages as those discussed above, with reference to US-3 982 538-A.

Accordingly, the present invention is directed to the provision of improved valve and/or filtering means which can be adapted for use in various types of applications including, particularly, aspirating systems of the type described above.

### Summary of the Invention

In accordance with the present invention, there is provided a non-mechanical porous valve, the internal passages through which are, prior to wetting with an aqueous liquid, sufficiently large in cross-sectional area to permit the flow of gas and liquid therethrough, and comprising :-
(A) a water insoluble resinous material swellable in response to wetting thereof by said aqueous liquid so as to close said passages substantially completely when said valve is wetted while the outer shape and gross volume of the valve is retained, thereby to prevent subsequent flow of gas and liquid through said valve; and
(B) a resinous filler;
which valve is characterised by a structure comprising the sintered product of 0.5 to 99 wt.% of a water insoluble, water absorbent resin capable of absorbing at least 50 g of water/g of resin, and 1 to 99.5 wt.% of a polyolefin which has a molecular weight of at least about 900,000 and which is capable of being sintered.

Species of the water insoluble, highly water-absorbent material used in the valve of the present invention are known and forms thereof are available commercially. For convenience, said material is hereafter often referred to as "highly water-absorbent material".

In preferred form, the device can also be made to serve as a filter, for example, to filter out contaminating living organisms such as certain types of bacteria, thereby combining the functions of a liquid-operable valve for gas and liquid and a biological filter. The preferred form of such a valve filter/valve device comprises a porous rigid structure of sintered particles of the water insoluble, highly water-absorbent material and filter particles, for example, polyethylene particles. The preferred highly water-absorbent material is a hydrolysed form of a graft copolymer prepared from starch and acrylonitrile, and is characterised by its ability to absorb at least about 100 parts of water per part of copolymer, and to maintain the absorbed water for a relatively long period of time, for example, at least about 4 hours.

It is theorized that the device functions in the following way. Upon contact with water, including, for example, aqueous body fluids, the particles of highly water-absorbent material substantially instantaneously absorb water and swell in a manner such that the spaces between the particles are filled, thereby rending the normally pervious or porous structure impervious to the flow of both air and liquid. In the preferred form of the invention, the aforementioned expansion or swelling of the particles is effected in a manner such that neither the outer shape of the valve nor its gross volume changes to a significant extent.

In the application of the invention in which the device is utilized normally to permit the flow of aspirating gas through it, but to block the flow of aspirated liquids through it as soon as it is wet by an aqueous liquid, it may be positioned along the flow path of aspirated air prior to the point at which the presence of collected liquid in or from the receptacle will be harmful or undesirable. In one preferred embodiment, the device is positioned within the collecting receptacle below the level of the top of the outlet from the receptacle, and in the path of the air which flows from inlet to outlet opening of the receptacle. For example, it may be placed at the lower end of a short tube which leads upwardly to the air outlet. In the latter case, when the level of the liquid in the receptacle rises sufficiently to contact the device, the passage through it of both air and liquid is prevented, thus placing the valve in its closed condition. It may also be located at other positions within the receptacle or even in an outlet line leading from the outlet of the receptacle to the vacuum source, if desired.

In one preferred embodiment, the receptacle may be in the form of a closed, air-tight and air-impervious plastic bag, the top of which is closed by a lid. The lid is sealed to the end of the bag and contains spaced-apart inlet and outlet air flow openings. The bag is adapted to be placed within a rigid cylindrical canister having an open top onto which the lid can be pressed to form an air tight seal therewith. A vacuum line leads from the receptacle outlet to the source of vacuum, and the inlet opening is connected by a patient tube to the site from which aqueous fluid is to be aspirated.

When the system is operating, the liquid is aspirated from the patient site through the patient tube and into the interior of the plastic bag, where it falls to the bottom and collects. Preferably, a connection is also provided between the vacuum source and the interior of the canister outside of the bag so that the pressure on the inner and outer sides of the bag can be equalized to prevent its collapse during operation. When the liquid has risen sufficiently to cover and wet the outer surface of the automatic flow-control device of the invention, no further flow of liquid or air will occur. The device is preferably positioned so that this will occur before the liquid has reached the lid of the receptacle and the outlet opening. If desired, an indicator and/or alarm device can be provided to produce an alarm indication to the operator that the receptacle is filled, using for this purpose, for example, the change in suction pressure in the vacuum line which will occur when gas flow has been cut off as described above. The flow control device will also function to cut off outlet flow from the receptacle if the receptacle is accidentally tipped over.

### Brief Description of Figures

Figure 1 is an idealized perspective view illustrating an application of the invention, in one of its preferred forms, to the aspirating of body liquids from a patient.

Figure 2 is a top plan view and Figure 3 is a side elevational view, with parts broken away, of a receptacle and canister arrangement in accordance with a preferred embodiment of the invention.

Figure 4 is an enlarged fragmentary view, in vertical section, of the top portion of the canister and receptacle assembly within the broken-line rectangle in Figure 3.

Figure 5 is a perspective view, with parts broken away, of one form of flow-controlling device in accordance with the invention, as used in the application of the invention shown in the foregoing drawings.

Figure 6 is a perspective view of a disc-shaped flow control device and/or filter according to the invention, as used in the embodiment of Figure 7.

Figure 7 is a fragmentary side elevational view, in section, showing a valve and/or filter assembly inside the receptacle and using the disc- shaped valve member of Figure 6 as the operative element.

And Figure 8 is a vertical sectional view showing a valve and/or filter assembly in series in the vacuum line and using the device shown in Figure 5.

### Detailed Description of the Invention

Without thereby in any way limiting the scope of the invention, it will now be described with specific reference to preferred embodiments of the device, of systems employing it, and of methods of its manufacture and use.

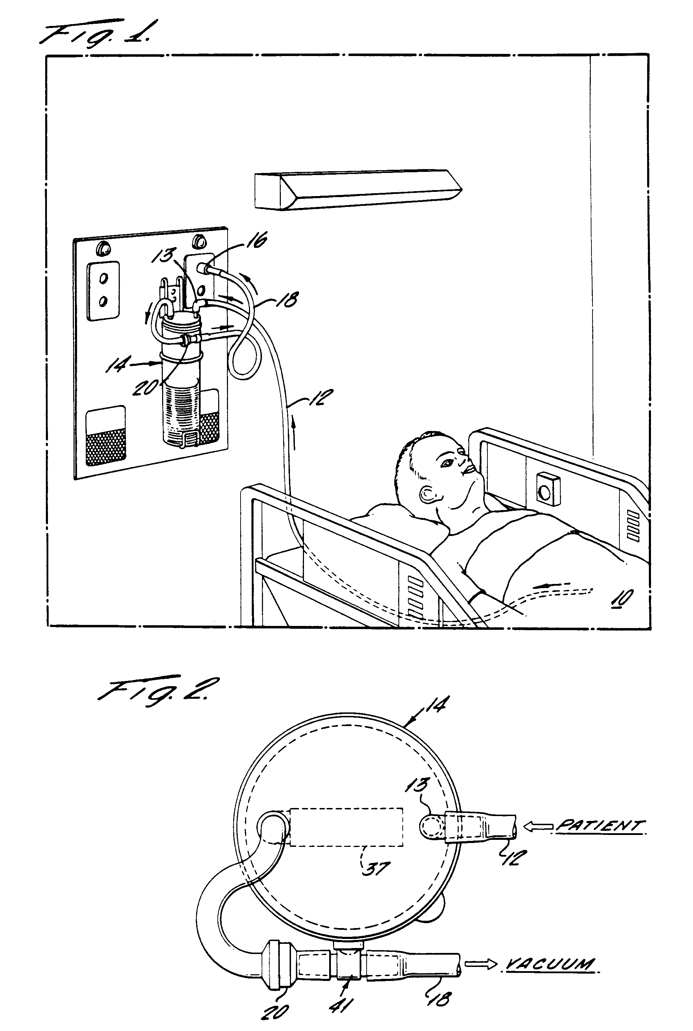

Figure 1 shows the site 10 at which a surgical operation has been performed upon a patient, and a patient tube 12 which leads from that site to the inlet fitting 13 to aspirate liquids, including any gases or small particles carried by the liquid, from the site of the operation to the nearby liquid-collecting system 14. A vacuum source 16, such as a hospital vacuum line, provides a suitable vacuum which normally acts through the vacuum line 18 and the liquid-collecting system 14 to produce suction at the free or aspirating end of the patient tube 12. An air check valve 20 may be included in series with the vacuum line. If desired, a suitable pinch valve can be provided near the aspirating end of the patient tube so that medical personnel may easily turn the aspirating action on and off at will.

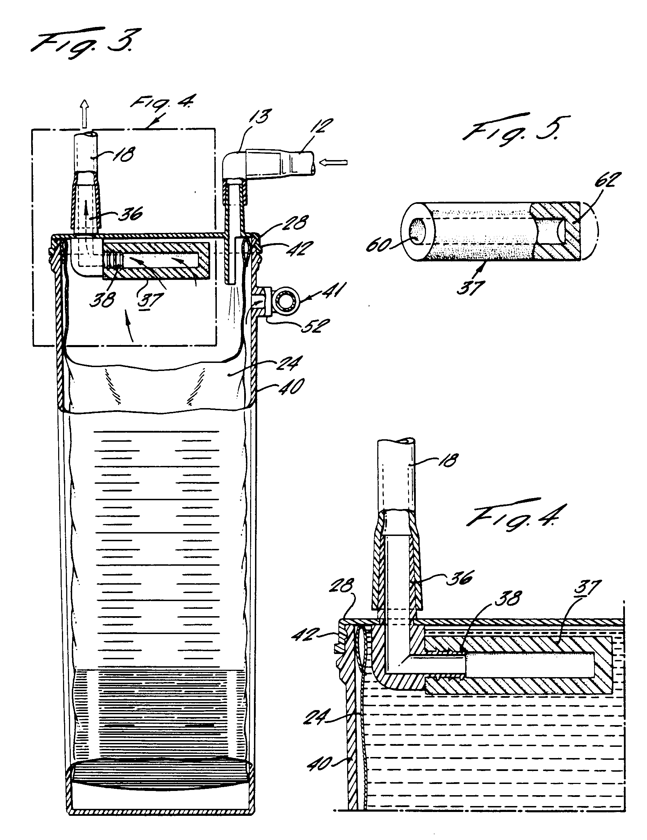

Referring now to Figures 2-5 for further details of the collecting system 14, there is shown a receptacle 24 in the form of a flexible plastic bag, the top opening of which is sealed to a lower surface of a solid plastic lid 28. The seal between receptacle 24 and lid 28 may be provided by any suitable air-impervious adhesive. The hollow, tubular, inlet fitting 13 extends through and above and below the lid, as does the hollow, tubular, outlet fitting 36.

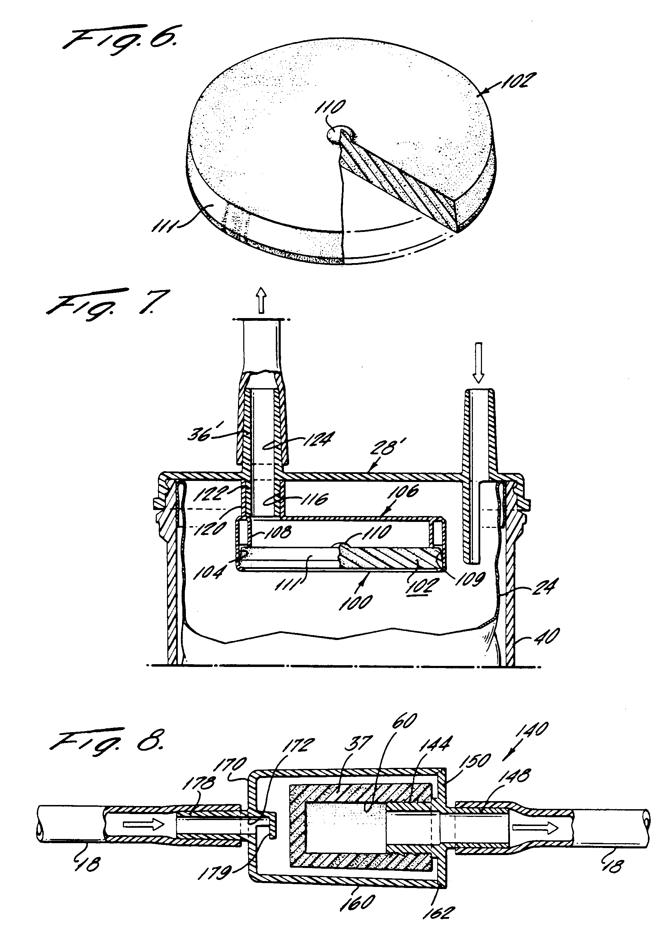

The flow control device 37 constructed in accordance with the invention is mounted on the end of outlet fitting 36 which lies within the receptacle 24, as by forcing it onto the nipple end 38 of the latter fitting, as shown more clearly in Figure 4.

The receptacle 24 is disposed within a canister 40, which may be of rigid plastic or metal and which provides mechanical support and protection for the bag assembly as well as support for a pressure-equalizing connector 41 used as described hereinafter to equalize the air pressure inside and immediately outside of the receptacle 24. It will be seen that a downwardly-extending rim portion 42 is provided on the lid 28 which can be readily snapped into air-tight relation with the top of the canister to complete the assembly of receptacle and canister and to enable later easy removal and discard of bag and lid. T-connector 41 provides communication at 52 between the vacuum line and the interior of the canister, so that as the vacuum line is evacuated, and the interior of the bag 24 therefore also evacuated, a correspondingly reduced air pressure is produced within the canister outside of the bag so the bag will not be subject to collapse during operation. The portion of the vacuum line 18 extending from the air outlet 36 on lid 28 is connected to the T-connector 41 as shown to complete the operative assembly.

During use then, after assembly of the system has been completed as described above, and the vacuum source is operating, liquids will be aspirated through the patient tube 12 and the inlet fitting 13 into the interior of the receptacle 24 where they will fall to the bottom of the bag and accumulate as shown in Figure 3. When they have accumulated to the level shown in Figure 4, the flow control device of the invention will be covered with the accumulated liquid, as a result of which it will become substantially impervious to the flow of gas or liquid through it. This therefore terminates the aspiration be fore the accumulated liquid reaches the level at which undesired overflow from the outlet fitting can occur. As mentioned above, suitable indicator or alarm means can be provided to indicate when this condition has been reached. The patient tube 12 can then be disconnected from the lid 28, the vacuum line 18 is disconnected from the outlet fitting 36, the lid 28 and the appended filled bag removed from the canister 40, and the lid and bag assembly discarded. It will therefore be seen that filling of the bag is automaticallly terminated as desired, without requiring the use of any moving parts or other complicated mechanism.

Figure 5 shows particular form for the filter and valve device itself, although it may take any of a large variety of different configurations in different applications. In this case, it is in the form of a cylinder which has a dead-ended central bore 60 providing an end wall 62, so that the aspirated air must pass through a wall of the device to reach the interior of the outlet fitting 36, which terminates inside bore 60 as shown in Figure 3. Although this form of the flow control device of the present invention is shown for use within the receptacle of an aspirating device, it can be used also in an aspirating device of the type in which the flow control device is located outside of the receptacle, for example, in an outlet line leading from the outlet of the receptacle to the vacuum source. Indeed, the form of the device shown in Figure 5 is preferred for use as an "external" flow control device, for example, as shown in Figure 8.

In Figure 8, the flow-control assembly 140 is shown placed in series in the vacuum line 18 which extends from the outlet fitting 36 to the vacuum source 16 in Figure 1. The flow device itself may be the same valve cylinder 37, with a central dead-ended bore 60, as is shown in Figure 5. In this example, a circumferentially-ribbed, hollow, tubular stem 144 extends snugly into the bore 60, and communicates with a central opening in the outlet tube 148 which is integral with stem 144. A circular flange 150, also integral with the stem and the tube, extends radially at the junction of stem and tube. An outer cylindrical casing 160 fits tightly about, and may be cemented to, a corresponding circular ledge 162 extending about the periphery of the flange, so that the outer cylindrical casing 160 surrounds the valve cylinder 37 at a radial distance therefrom. The opposite end of the outer cylindrical casing is closed by a circular end wall 170 having a central opening 172 communicating with the interior of an inlet tube 178 which extends outwardly therefrom. Inlet tube 178 also extends slightly into the outer cylindrical casing 160, is closed at its interior end, and has an opening 179 in its sidewall adjacent its interior end, so that the flow of air into the casing 160 will be diffused and distributed, rather than concentrated on the adjacent end of the valve cylinder. All parts of the assembly may be made of a transparent polyacrylic material, as an example.

Upon application of vacuum, air or other gases from the upstream end of the vacuum line 18 will flow through inlet tube 178 to the interior of the plastic outer cylindrical casing 160, then through the wall of valve 37 to its central bore, and finally out of tube 148 to the downstream portion of vacuum line 18. If collected liquid should flow from the receptacle 24 into the vacuum line 18, when the liquid reaches valve 37 the valve will become impervious to further flow of liquid or gas, as described previously, thus preventing flow of either type of fluid further in to the vacuum line and system, as desired.

Figure 6 shows a particularly preferred form of a flow control device for use within the receptacle of an aspirating device, for example, as shown in the embodiment of Figure 7, in which the flow control assembly 100 is located inside the receptacle 24. This assembly uses a valve in the form of the disc 102 of Figure 6, which is pressed into a circular recess 104 in the underside of the disc-supporting cup 106 until a marginal area of the upper side of the disc bears against the bottom of the internal ring-like band 108 in cup 106, to form a seal therewith; the internal sidewalls 109 of the lower portion of the cup are tapered slightly inwardly along the upward direction, and the disc is inserted with the boss 110 positioned upwardly so that the inwardly-slanted portions 111 of the edge of the disc mate with the inwardly-slanted internal sidewalls of the cup 106. The disc may be solvent-welded to the inner side walls of the cup into which it is thus placed. A plastic retaining ring 112 may then be formed by heat-knurling the bottom end of the cup to bridge the space between the bottom of the cup and the marginal portions of the lower side of the disc, thereby to secure the disc positively in place.

The cup is also provided with a top opening 116 communicating with an integral upwardly-extending tube 120. The latter tube is slipped over the outside of a downwardly-depending hollow nipple 122 formed integrally on the underside of lid 28', and is cemented or otherwise secured thereto. The opening in the nipple is aligned with an opening 124 extending through the lid 28' and communicating with the interior of the upwardly-extending tubular outlet fitting 36'. When fluid in the receptacle rises to the level at which the surfaces of disc 100 are wetted, further flow of air or gas is automatically terminated as described previously.

The flow control device of the present invention comprises a material which is capable of absorbing a relatively large amount of water, for example, at least about 50 parts by weight of water per part by weight of material. For use in collecting systems of the type described above, the highly water-absorbent material should resist being affected adversely as a result of contact with blood, body fluids, medications and other materials which may be encountered in such an aspirating application. In addition, the highly water-absorbent material should not be a source of contamination of the material which is collected in the receptacle of the collecting system. In situations where it is desired to analyze the collected materials, contamination thereof cannot be tolerated.

Presently, species of water insoluble, highly water-absorbent materials which can be used in the practice of the present invention comprise synthesized resins, including, for example, chemically treated naturally occurring polymeric materials and synthetic polymeric materials prepared by polymerization of monomers. An exemplary class of materials prepared from chemically treating naturally occurring polymeric materials comprise those which are synthesized from naturally occurring polymeric materials such as, for example, carboxymethylcelluloses, polysaccharides such as, for example, starch, and natural gums such as, for example, guar gum, and from vinyl compounds which are reactive therewith.

As mentioned above, species of the water insoluble, highly water-absorbent material comprising the flow-control device of the present invention are known. The following U.S. patents disclose examples of water-absorbent materials and processes for preparing the same: Nos. 3,425,971; 3,661,815; 3,997,484; 4,076,663 and 4,302,369. Speaking generally, the aforementioned patents are related to graft copolymers which are prepared from starch and acrylonitrile. Their disclosures are incorporated herein by reference with respect to the polymers described therein and the processes for preparing the polymers.

As set forth in aforementioned U.S. Patent No. 3,425,971, the water-absorbent polymer described therein is prepared by hydrolyzing a starch/polyacrylonitrile graft copolymer, the hydrolysis being effected by treating the polymer with an aqueous solution of an alkaline material or base, for example, potassium hydroxide to form a carboxylate. (The hydrolyzed form of the graft copolymer is characterized as containing both carboxyl and amide groups.) Addition of acid to the carboxylate forms the free acid which in turn can be precipitated from an alcohol solution. Aforementioned U.S. Patent No. 3,661,815 discloses that the water-absorbing properties of resins disclosed in the aforementioned '971 patent are reduced upon contact with urine, and that this problem can be overcome by conducting the aforementioned type of hydrolytic reaction or saponification with an alkaline solution that includes alcohol. Aforementioned U.S. Patent No. 3,997,484 discloses that the water-absorbing properties of a hydrolyzed graft starch/polyacrylonitrile resin or starch/polymethacrylonitrile can be increased by using gelatinizing starch to prepare the graft copolymer. Insoluble resin can be obtained by evaporating the aqueous alkaline solution that is used in the hydrolytic or saponifying step. Aforementioned U.S. Patent No. 4,076,663 discloses the preparation of a water-absorbent resin prepared by polymerizing the following monomers: (A) starch or cellulose; (B) at least one monomer having a polymerizable double bond which is water-soluble or becomes water-soluble by hydrolysis; and (C) a cross-linking agent; and, if necessary, subjecting the resulting product to hydrolysis. Aforementioned U.S. Patent No. 4,302,369 discloses that problems are encountered when highly water-absorbent resins are subjected to water which contains free ions, for example, sodium, and that such problems can be overcome by reacting the hydrolyzed form of a starch or gelatinized starch/acrylonitrile graft copolymer with aluminum to produce an aluminum salt of the copolymer. Such resins are said to have an enhanced degree of wicking and over-all improved water-absorbing properties.

Exemplary water-absorbing properties of resins which are the subjects of said patents have the capability of absorbing about 50 to about 1000 parts or more of water per part of resin. Such resins can be produced in water-insoluble granular form and have the ability to absorb such large amounts of water while retaining their granular form. Various of the aforementioned patents disclose that water-absorbing resins of the type disclosed therein can be used in many types of applications including, for example, as water or body-fluid absorbents in diapers, sanitary napkins, bed or surgical pads, and disposable dust cloths, and to increase the water-holding capacity of soils, and as coatings for seeds, clay, starch, fibers, paper and the like. In addition, U.S. Patent No. 4,421,129 discloses the use of such resins in preparing shaped articles which can be used to rapidly dry hair. Examples of such articles include combs, hair brushes, and hair curlers which are made by molding a powdery form of the highly water-absorbent polymer in a mold under pressure and at ambient temperature. Such articles take advantage of those proper ties of the water-absorbent resin by which large quantities of water are quickly absorbed in a short time, for example, within minutes, without causing a change in the gross shape of the article. The rate at which absorbed water evaporates from the resin varies depending on the resin, the evaporation rate for some resins being substantially the same as that of water of similar surface area. As water evaporates from the resin, it is capable of being reused to absorb water.

It should be understood that water insoluble, highly water-absorbent materials other than the graft starch polymers mentioned above can be used in the practice of the present invention. Examples of such resins include cross-linked poly(alkylene oxide) resins, cross-linked polyacrylate resins, and cross-linked carboxy methylcellulose resins. Species of such resins are known and include materials which are available commercially. The following U.S. patents disclose examples of additional species of water-absorbent resins: Nos. 3,264,202; 3,898,143 and 3,956,224.

Examples of commercially available water-absorbent resins include the following trademark products sold by Grain Processing Corporation: Water-Lock A-100 characterized as being insoluble in most organic solvents, resistant to body fluid enzymes and capable of absorbing dilute aqueous acid and alkali solutions, and having the ability to absorb at least 110 ml of water/g of resin; Water-Lock J-500 characterised as being capable of absorbing at least about 500 ml of water/g of resin; and Water-Lock J-550 characterised as being capable of absorbing at least 375 ml of water/g of resin. Such resins are described by their manufacturer as being super absorbent products which are prepared by hydrolyzing starch/acrylonitrile graft copolymers to form polymeric products having side chains which contain carboxamide and carboxylate groups. The aforementioned resins are available commercially as free flowing powders. Other sources of the resins include Arakawa Chemical (U.S.A.) Inc. which sells their resins under the trademark "ARASORB", Sanyo Chemical, Unilever, Hercules, and Dow Chemical.

The flow control device of the present invention can be made almost entirely (and up to 99% by weight) from the water insoluble, highly water-absorbent material, together with one or more other materials, including major amounts (up to 99.5% by weight) of such other materials. From 1 to 99.5% of a polyolefin having a molecular weight of at least about 900,000 and which is capable of being sintered is included, but so too may be other materials such as carriers and additives which improve the physical and/or chemical properties of the composition comprising the device. In this regard, the composition can comprise, for example, polyethylene, acrylic resin, and nylon (to name but a few), a filler material such as calcium carbonate, lubricant, anti-oxidant, fibers, including both synthetic and natural fibers, and materials which modify one or more of the asthetic, physical and chemical properties of the composition, for example molybdenum disulfide. The polyolefin filler may comprise a polyethylene or a polypropylene.

With regard to a flow control device for use in an as pirating system of the type described above, particularly good results have been achieved by fabricating such device from a mixture of the highly water absorbent resin and of water insoluble polymeric carrier or filler particles.

An attractive feature of the present invention is that relatively small amounts of the highly water-absorbent resin can be used in fabricating the flow-control device. Thus, a minor amount of the resin can be combined with a major amount of the carrier or filler material(s), including material(s) which is less costly than the water-absorbent resin. In addition to cost advantages, the strength of the device can be improved by the use of a carrier material, preferred materials being polyethylenes, and polypropylenes having a molecular weight of at least about 900,000 to several million. (Lower molecular weight polyolefins whose molecular weight can be increased by chemical modification such as, for example, irradiation and peroxide treatment of the polymer, can be used also.) It is preferred also that the melt index of such polymeric materials be less than 1. Based on cost, ease of manufacture, and performance, polyethylenes having a molecular weight of at least about 2.5 million and a melt index below 1 are particularly desirable material for use in the present invention. Recommended commercial products are those sold under the trademark HOSTALEN GUR and HIMONT, for example HOSTALEN GUR 412, 413, and 415, and HIMONT HB 312.

Accordingly, the amount of water-absorbent resin comprising the fluid-control device can vary over a wide range, the minimum amount being dictated by that needed to block the passage of the aqueous fluid through the device as it is contacted therewith, and the maximum amount being about 100 wt.% of the resin. For example, a device within the present invention can comprise about 0.5 to about 99 wt.% of the water-absorbent resin and about 1 to about 99.5 wt.% of filler. Preferred amounts of constituents comprise about 5 to about 15 wt.% resin and about 85 to about 95 wt.% filler.

The device of the present invention can be made in various ways, including, for example, by the method disclosed in aforementioned U.S. Patent No. 4,421,129 for fabricating the hair drying devices described therin. This method involves basically forming the article by injection or compression molding techniques utilizing elevated pressures and ambient temperature. For example, powdered resin is placed into a hardened steel mold and formed into an article of the desired shape at pressures of about 200 to about 40,000 psi at ambient temperature and within one minute. Elevated temperatures can be used also in the molding operation.

### Examples

Examples which follow are illustrative of the fabrication of fluid-control devices within the scope of the present invention.

### Example 1

A fluid-control device as shown in Figure 5 was made in the following manner. The device had an overall length of 2.1 inches, a bore length of 1.9 inches, an overall diameter of 0.69 inch and a bore diameter of 0.295 inch.

The device was fabricated from a composition comprising: (A) 90 wt.% of powdered polyethylene having a molecular weight of about 3.5 million, a melt index below 1, and a spherical particle size diameter of about 50 microns; and (B) 10 wt.% of highly water-absorbent resin particles comprising a hydrolyzed form of a starch/acrylonitrile copolymer sold by Grain Processing Corporation under the trademark Water-Lock A-200.

The resin and polyethylene particles were ball-milled to produce a mixture in which particles of the water-absorbent resin were adhered to the larger size particles of polyethylene. The resin mixture was placed into an approximately shaped mold and then made into the fluid-control device by compression molding at a temperature of about 375°F and a pressure of about 1000 psi, and for a time of about 10-15 minutes.

The device had a pore size of about 5 microns. Evaluating work showed that the device was capable of: (A) filtering 99.8% of bacteria as small as Pseudomonas diminutae and 100% of larger bacteria; and (B) holding fluids of the type collected in the receptacle of the collecting system described above for in excess of 8 hours.

In use in an aspirating system of the type described above, the fluid-control device of Examle 1 immediately upon being wetted with the liquid contents of the receptacle prevented passage thereof beyond the device. It has been observed that foam generated from materials such as blood, soap and egg whites do not adversely affect the function of the device, nor does it cause a slow down or premature termination of air flowing there through. It has been observed also that, in use, the flow-control device of Example 1 permitted the passage therethrough of laser plume with minimal air flow restrictions. The device was used in an application which included an initial air flow in the range of about 3.6 to about 5 cubic ft/min and with air flow at the patient site being no less than about 2.6 cubic ft/min, a satisfactory value.

### Example 2

A flow-control device as shown in Figure 5 and described in Example 1 is made, as described in Example 1, but with somewhat different dimensions, the device being contemplated for use as an "external" filter/valve in an aspirating device. The device has an overall length of 1.7 inches, a bore length of 1.5 inches, an overall diameter of 1 inch and a bore diameter of 0.6 inch. The flow-control device has a uniform porosity of about 9.75 micron pore radius and a pore volume of about 50%. It is capable of operating at a source vacuum of about 3 SCFM (24'' Hg) with an air flow of no less than about 2.5 SCFM - even when exposed to laser plume for 2 hours. Under use conditions, the flow-control device is capable also of not permitting any fluid to pass through it for at least 8 hours. Filtering characteristics include the ability of filtering more than 99% of Pseudomonas diminutae and 100% of Micrococcus luteus and B. subtilis.

### Example 3

A flow control device in the form of a disc, as shown in Figure 6, is made from a composition like that described in Example 1 and in the manner described in Example 1. The diameters of the lower and upper surfaces of the disc are respectively 2.43 and 2.40 inches. The thickness of the disc is 0.2 inch. The inwardly slanted portions of the edge of the disc are inclined at an angle of 5°, with the slanted portions beginning at a distance of 0.05 inch from the lower surface of the disc. The porosity and pore volume of the device are like those of the device of Example 2 and its operating characteristics are also like those of the device of Example 2.

The embodiments described above are shown for use in apparatus for aspirating body fluids from the site of a body wound. The apparatus which is illustrated functions by virtue of the use of reduced pressure or vacuum which draws liquid away from the body and into the apparatus. Devices within the scope of the present invention can be used also in other types of applications involving the use of a vacuum pump to protect the pump from undesired contact with fluids. It should be understood that devices within the scope of the present invention can be used also in other types of applications, including, for example, applications in which gas flow through the device is under positive pressure.

## Claims

1. A non-mechanical porous valve, the internal passages through which are, prior to wetting with an aqueous liquid, sufficiently large in cross-sectional area to permit the flow of gas and liquid therethrough, and comprising:-
(A) a water insoluble resinous material swellable in response to wetting thereof by said aqueous liquid so as to close said passages substantially completely when said valve is wetted while the outer shape and gross volume of the valve is retained, thereby to prevent subsequent flow of gas and liquid through said valve; and
(B) a resinous filler;
which valve is characterised by a structure comprising the sintered product of 0.5 to 99 wt.% of a water insoluble, water absorbent resin capable of absorbing at least 50 g of water/g of resin, and 1 to 99.5 wt.% of a polyolefin which has a molecular weight of at least about 900,000 and which is capable of being sintered.

2. A valve according to claim 1, characterised in that said polyolefin consists essentially of polyethylene or polypropylene having a melt index below 1.

3. A valve according to claim 2, characterised in that said polyolefin consists essentially of polyethylene which has a molecular weight of at least about 2.5 million.

4. A valve according to claim 3, characterised in that said structure comprises 5 to 15 wt.% of said resin and 85 to 95 wt.% of said polyethylene.

5. A valve according to any of claims 1 to 4, characterised in that said water-absorbent resin is capable of absorbing at least 100 parts of water/part of resin and of maintaining the absorbed water for at least 4 hours.

6. A composition capable of being sintered and formed into the valve of any of claims 1 to 5, characterised by the unsintered mixture of said water insoluble, water absorbent resin and said polyolefin.

7. A valve system for automatically controlling the flow of liquid through it comprising:
(A) the valve of any of claims 1 to 5;
(B) means for supplying gas at a first pressure to one region of said valve while maintaining a second lower gas pressure at another region of said valve; and
(C) means for mounting said valve in a position in which the portion therefore between said one and said another regions thereof is subject to wetting by an aqueous liquid.

8. A valve system according to claim 7, characterised in that said cross-sectional areas of the passages of the valve, prior to wetting, are sufficiently small for said valve to act as a bacterial filter.

9. A valve system according to claim 9, characterised in that the size of said passages is such that bacteria having the size of Pseudomonas dimunutae bacteria and bacteria of larger sizes are prevented from passing through said valve.

10. A valve system according to any of claims 7 to 9, characterised in that said polyolefin consists essentially of polyethylene having a molecular weight of at least 2.5 million.

11. An aspirating system for removing liquids from the vicinity of a living body, comprising a vacuum source, an aspirator tube one end of which is adapted to be placed in the immediate vicinity of said body to aspirate liquids therefrom, a liquid-collecting receptacle having an inlet and an air outlet, first means connecting the other end of said aspirator tube to said inlet, a vacuum line operatively connecting said vacuum source to said air outlet, said inlet and said outlet both being spaced from the bottom of said receptacle whereby said receptacle may be filled to a substantial depth with said liquid before the level of said liquid reaches said inlet or said outlet; there being means for preventing said collected liquid from passing out of said air outlet of said receptacle and beyond a predetermined point in said vacuum line, which means is a normally gas- and liquid-pervious flow-control member positioned in the path of the collected liquid between a point in the interior of said receptacle and said predetermined point in said vacuum line, characterised in that said flow-control member comprises the valve of any of claims 1 to 5.

## Patentansprüche

1. Nicht-mechanisches poröses Ventil, dessen innere Durchgangswege vor dem Befeuchten mit einer wasserhaltigen Flüssigkeit in der Querschnittsfläche genügend groß sind, um den Durchfluß von Gas und Flüssigkeit zuzulassen, mit:
(A) einem wasserunlöslichen, harzartigen Material, das in Reaktion auf dessen Befeuchtung durch die wasserhaltige Flüssigkeit schwellbar ist, um so die Durchgänge im wesentlichen vollständig zu schließen, wenn das Ventil befeuchtet ist, während die äußere Form und das Gesamtvolumen des Ventils beibehalten werden, um dadurch einen nachfolgenden Fluß von Gas und Flüssigkeit durch das Ventil zu verhindern; und
(B) einem harzartigen Füllstoff;
wobei das Ventil gekennzeichnet ist durch eine Struktur mit dem gesinterten Produkt von 0,5 bis 99 Gew.% eines wasserunlöslichen, wasserabsorbierenden Harzes, das in der Lage ist, wenigstens 50 Gramm Wasser pro Gramm Harz zu absorbieren, und 1 bis 99,5 Gew.% eines Polyolefins, das ein Molekulargewicht von wenigstens etwa 900.000 hat und sinterbar ist.

2. Ventil nach Anspruch 1, dadurch gekennzeichnet, daß das Polyolefin im wesentlichen aus Polyethylen oder Polypropylen mit einem Schmelzindex unterhalb von 1 besteht.

3. Ventil nach Anspruch 2, dadurch gekennzeichnet, daß das Polyolefin im wesentlichen aus Polyethylen mit einem Molekulargewicht von wenigstens etwa 2,5 Millionen besteht.

4. Ventil nach Anspruch 3, dadurch gekennzeichnet, daß die Struktur 5 bis 15 Gew.% des Harzes und 85 bis 95 Gew.% des Polyethylens aufweist.

5. Ventil nach einem der Ansprüche 1 bis 4, dadurch gekennzeichnet, daß das wasserabsorbierende Harz in der Lage ist, wenigstens 100 Teile Wasser pro Teil Harz zu absorbieren und das absorbierte Wasser für wenigstens vier Stunden zu halten.

6. Zusammensetzung, die sinterbar und zu dem Ventil nach einem der Ansprüche 1 bis 5 formbar ist, gekennzeichnet durch die ungesinterte Mischung des wasserunlöslichen, wasserabsorbierenden Harzes und des Polyolefins.

7. Ventilsystem zum automatischen Steuern des Flusses an Flüssigkeit dort hindurch, mit:
(A) dem Ventil nach einem der Ansprüche 1 bis 5;
(B) einer Einrichtung zum Liefern von Gas bei einem ersten Druck zu einem Bereich des Ventils, während an einem anderen Bereich des Ventils ein zweiter, niedrigerer Gasdruck gehalten wird; und
(C) einer Einrichtung zum Montieren des Ventils in einer Position, in der der Teil davon zwischen dem einen und dem anderen Bereich einer Befeuchtung durch eine wasserhaltige Flüssigkeit ausgesetzt ist.

8. Ventilsystem nach Anspruch 7, dadurch gekennzeichnet, daß die Querschnittsflächen der Durchgänge des Ventils vor dem Befeuchten ausreichend klein sind, damit das Ventil als ein Bakterienfilter wirken kann.

9. Ventilsystem nach Anspruch 8, dadurch gekennzeichnet, daß die Größe der Durchgänge derart ist, daß Bakterien mit der Größe von Pseudomonas dimunutae-Bakterien und Bakterien mit größeren Größen an einer Passage durch das Ventil gehindert sind.

10. Ventilsystem nach einem der Ansprüche 7 bis 9, dadurch gekennzeichnet, daß das Polyolefin im wesentlichen aus Polyethylen mit einem Molekulargewicht von wenigstens 2,5 Millionen besteht.

11. Absaugsystem zum Entfernen von Flüssigkeiten aus der Umgebung eines lebenden Körpers, mit einer Unterdruckquelle, einem Saugrohr, wovon ein Ende dazu ausgelegt ist, in der unmittelbaren Nachbarschaft des Körpers angeordnet zu werden, um Flüssigkeiten davon abzusaugen, einem Gefäß zum Sammeln von Flüssigkeiten mit einem Einlaß und einem Luftauslaß, ersten Mitteln zum Verbinden des anderen Endes des Saugrohres mit dem Einlaß, einer Unterdruckleitung, die die Unterdruckquelle mit dem Luftauslaß betriebsfähig verbindet, wobei der Einlaß und der Auslaß beide auf Abstand von dem Boden des Gefäßes angeordnet sind, wodurch das Gefäß auf eine beträchtliche Tiefe mit der Flüssigkeit gefüllt werden kann, bevor der Füllstand der Flüssigkeit den Einlaß oder den Auslaß erreicht; einer Einrichtung zum Verhindern, daß die gesammelte Flüssigkeit aus dem Luftauslaß des Gefäßes und hinter einen vorbestimmten Punkt in der Unterdruckleitung gelangt, welche Einrichtung ein normalerweise gas- und flüssigkeitsdurchlässiges Flußsteuerungsbauteil ist, das im Weg der gesammelten Flüssigkeit zwischen einem Punkt im Inneren des Gefäßes und dem vorbestimmten Punkt in der Unterdruckleitung angeordnet ist, dadurch gekennzeichnet, daß das Flußsteuerungsbauteil das Ventil nach einem der Ansprüche 1 bis 5 aufweist.

## Revendications

1. Valve poreuse non-mécanique, dont les passages internes qui la traversent ont, avant mouillage par un liquide aqueux, une aire de section transversale suffisamment grande pour permettre l'écoulement de gaz et de liquide à travers ceux-ci, et comprenant :
A - un matériau résineux insoluble dans l'eau, capable de gonfler en réponse à son mouillage par ledit liquide aqueux de façon à fermer sensiblement complètement les passages lorsque la valve est mouillée tandis que la forme extérieure et le volume brut de la valve sont conservés, de façon à empêcher ensuite l'écoulement de gaz et de liquide à travers la valve ; et
B - une charge résineuse ;
la valve étant caractérisée par une structure comprenant le produit fritté formé de 0,5 à 99 % en poids d'une résine insoluble dans l'eau, absorbant l'eau, et capable d'absorber au moins 50 g d'eau/g de résine, et 1 à 99,5 % en poids d'une polyoléfine ayant un poids moléculaire d'au moins environ 900 000 et qui est capable d'être frittée.

2. Valve selon la revendication 1, caractérisée en ce que ladite polyoléfine consiste essentiellement en du polyéthylène ou polypropylène ayant un indice de fusion inférieur à 1.

3. Valve selon la revendication 2, caractérisée en ce que ladite polyoléfine consiste essentiellement en un polyéthylène ayant un poids moléculaire d'au moins 2,5 millions.

4. Valve selon la revendication 3, caractérisée en ce que ladite structure comprend 5 à 15 % en poids de ladite résine et 85 à 95 % en poids dudit polyéthylène.

5. Valve selon l'une quelconque des revendications 1 à 4, caractérisée en ce que ladite résine absorbant l'eau est capable d'absorber au moins 100 parties d'eau/partie de résine et de conserver l'eau absorbée pendant au moins quatre heures.

6. Composition capable d'être frittée et formée en la valve de l'une quelconque des revendications 1 à 5, caractérisée par le mélange non fritté de ladite résine insoluble dans l'eau et absorbant l'eau, et de ladite polyoléfine.

7. Système de valve pour commander automatiquement l'écoulement qui la traverse, comprenant :
A - la valve de l'une quelconque des revendications 1 à 5 ;
B - des moyens pour fournir du gaz à une première pression en une première région de ladite valve tout en maintenant une seconde pression de gaz, plus basse, en une autre région de ladite valve ; et
C - des moyens pour monter ladite valve dans une position dans laquelle la partie de celle-ci entre lesdites première et autre régions de celle-ci est soumise à mouillage par un liquide aqueux.

8. Système de valve selon la revendication 7, caractérisé en ce que lesdites aires de section transversale des passages de la valve, avant mouillage, sont suffisamment petits pour que cette valve serve de filtre bactériologique.

9. Système de valve selon la revendication 9, caractérisé en ce que la taille desdits passages est telle que des bactéries ayant la taille de bactéries Pseudomonas dimunutae et de bactéries de plus grande taille sont empêchées de traverser la valve.

10. Système de valve selon l'une quelconque des revendications 7 à 9, caractérisé en ce que ladite polyoléfine consiste essentiellement en un polyéthylène ayant un poids moléculaire d'au moins 2,5 millions.

11. Système d'aspiration pour ôter des liquides du voisinage d'un corps vivant, comprenant une source de vide, un tube aspirateur dont une première extrémité est adaptée à être placée dans le voisinage immédiat dudit corps pour en aspirer des liquides, un réceptacle collecteur de liquide ayant une sortie d'air et une entrée, des premiers moyens reliant l'autre extrémité du tube aspirateur à ladite entrée, une ligne d'aspiration reliant fonctionnellement ladite source de vide à ladite sortie d'air, ladite entrée et ladite sortie étant toutes deux écartées du fond du réceptacle de sorte que le réceptacle peut être empli dudit liquide jusqu'à une hauteur substantielle avant que le niveau de ce liquide atteigne ladite entrée et ladite sortie ; des moyens étant prévus pour empêcher le liquide collecté de sortir de ladite sortie d'air du réceptacle et d'aller au-delà d'un point prédéterminé dans la ligne d'aspiration, ces moyens étant un organe de commande d'écoulement normalement perméable aux gaz et aux liquides, positionné dans le trajet du liquide collecté entre un point à l'intérieur du réceptacle et ledit point prédéterminé dans la ligne d'aspiration, caractérisé en ce que ledit organe de commande d'écoulement comprend la valve de l'une quelconque des revendications 1 à 5.
